# EUROPEAN PATENT APPLICATION

(11) **EP 4 053 854 A1**
(43) Date of publication of application: **07.09.2022**
(21) Application number: 19950637.9
(22) Date of filing: 31.10.2019
(51) Int. Cl.: G16H 80/00

(54) **NURSING SUPPORT SYSTEM AND REPORT GENERATING DEVICE**

(71) Applicant: Hitachi, Ltd., Tokyo 100-8280 (JP)
(72) Inventor: MIYATA, Tatsuhiko, Beijing 100190 (CN); LIN, XiaoLie, Beijing 100190 (CN); YAMANAKA, Eiji, Beijing 100004 (CN); MIYAZAKI, Kunihiko, Tokyo 100--8280 (JP); UCHIDA, Makio, Tokyo 100-8280 (JP); FU, Ziyi, Beijing 100004 (CN); MATSUMORI, Masaki, Beijing 100190 (CN)
(74) Representative: MERH-IP Matias Erny Reichl Hoffmann Patentanwälte PartG mbB
(86) International application number: PCT/CN2019/114642
(87) International publication number: WO 2021/081874

(57) **Abstract**

A nursing support system and a report generating device are disclosed. The nursing support system includes: a detection device for detecting the body of a nursing recipient to obtain detection data related to the physical status of the nursing recipient; a first report generating device for generating, according to the detection data, evaluation indexes based on international evaluation standards, i.e., international evaluation standard data, and generating a nursing report for the nursing recipient by analyzing the international evaluation standard data, the international evaluation standards being standards for evaluating human health status; and an output device for outputting the nursing report.

## Description

### Technical Field

The present invention relates to a nursing support system and a report generating device.

### Background Art

At present, the society is gradually entering an aging society, and the nursing for the aged, i.e., nursing recipients, provided by nursing institutions such as aged care institutions has attracted more and more attention.

It is very important for a nursing institution to accurately grasp the health status of nursing recipients when nursing them.

In the past, as disclosed in Patent Literature 1, the health status of nursing recipients is known by questionnaires, for example, asking whether they can participate in a specific exercise, whether they can participate in some kinds of trainings to improve their athletic abilities, etc.

### Prior Art Document

### Patent Literature

Patent Literature 1: Japanese Patent Laid-Open No. 2017-211683

### Summary of Invention

### Technical Problem

However, in the replies to such questionnaires, a subjective consciousness of nursing recipients is inevitably involved, and the nursing recipients in the same health status may provide different answers, resulting in deviations and failure to accurately grasp the health status of the nursing recipients.

In addition, the method of questionnaire cannot predict the trend of the future health status of a nursing recipient.

Since it is impossible to accurately grasp the current health status of the nursing recipient and predict the trend of the future health status, it is impossible to create a nursing plan suitable for the nursing recipient.

The present application is proposed in view of the above circumstances, and the objective of the present application is to provide a nursing support system, which can accurately grasp the health status of a nursing recipient, predict a trend of the health status, and create a nursing plan suitable for the nursing recipient. Solution to Technical Problem
- Technical solution 1 is a nursing support system, comprising:
   a detection device for detecting the body of a nursing recipient to obtain detection data related to the physical status of the nursing recipient;
   a first report generating device for generating, according to the detection data, evaluation indexes based on international evaluation standards, i.e., international evaluation standard data, and generating a nursing report for the nursing recipient by analyzing the international evaluation standard data, the international evaluation standards being standards for evaluating human health status; and
   an output device for outputting the nursing report.

In the nursing support system of the present invention, the detection data obtained by the detection device are all objective data, so the international evaluation standard data generated according to these objective detection data can objectively and accurately reflect the health status of the nursing recipient. Therefore, the nursing staff can accurately grasp the health status of the nursing recipient, and provide targeted nursing services to the nursing recipient accordingly.
- The nursing support system of technical solution 2,
   further comprising a second report generating device, which analyzes the international evaluation standard data, management status data indicating the management status of a nursing institution, and insurance data indicating a health insurance that the nursing recipient is enrolled in, to generate a management report suitable for the nursing institution.

Since the international evaluation standard data, the management status data, and the insurance data are all objective data, objective management reports can be provided for the manager of the aged care institution, to accurately grasp the current management status and make a future management plan of the aged care institution according to the current management status and the status of the nursing recipient.
- The nursing support system of technical solution 3, wherein the first report generating device includes:
   a first memory for storing the detection data; and
   a first data analysis device for generating the international evaluation standard data according to the detection data, and generating the nursing report by analyzing the international evaluation standard data,
   wherein the second report generating device includes:
      a second memory for storing the management status data and the insurance data; and
      a second data analysis device for generating the management report by analyzing the international evaluation standard data input by the first data analysis device, the management status data, and the insurance data.
- The nursing support system of technical solution 4, wherein the nursing report includes a current health status report indicating the current health status of the nursing recipient and/or a future health status report predicting a trend of the future health status of the nursing recipient.

The nursing support system of the present invention can not only grasp the past health status of the nursing recipient, but also know the trend of the future health status, and therefore can reasonably arrange future nursing plans such as health training.
- The nursing support system of technical solution 5, wherein the current health status report is a training report of the nursing recipient on the current day, including information on brain activity test scores and walking ability test scores of the nursing recipient, information on the ranking of brain activity and walking ability among all nursing recipients, mean value comparison with contemporaries, and individual detailed training scores.
- The nursing support system of technical solution 6, wherein the future health status report is a predictive analysis report of detection data of the nursing recipient in a future prescribed period, including information indicating a brain activity trend, a walking ability trend, a summary of analysis results, and a recommended training menu for the nursing recipient.
- The nursing support system of technical solution 7, wherein the management report includes a current management report indicating the current management status of the nursing institution and/or a future management report predicting the future management status of the nursing institution, the current management report includes current financial information and distribution information of current nursing levels required by the nursing recipients, and the future management report includes future financial information and distribution information of future nursing levels required by the nursing recipients.

Thus, the man-hours required for the manager of the aged care institution to predict the future management status can be reduced.
- The nursing support system of technical solution 8,
   wherein the detection device includes a detection tool for detecting the body of the nursing recipient, and the detection tool detects the body of the nursing recipient to obtain detection data related to the physical status of the nursing recipient; the detection data includes test data, vital sign data and basic data; the test data includes at least one of computing ability scores, Oxy-HB (oxyhemoglobin) variation, walking speed, average stride and average acceleration; the vital sign data includes at least one of maximum blood pressure, minimum blood pressure, pulses, and blood glucose level; the basic data includes at least one of gender, age, height, and weight,
   wherein the international evaluation standards generated by the first report generating device include at least one of MMSE (Minimum Mental State Examination), FAB (Frontal Assessment Battery), TMT (Trail Making Test), and TUG (Timed Up & Go Test),
   wherein the nursing report includes a current health status report indicating the current health status of the nursing recipient and/or a future health status report predicting a trend of the future health status of the nursing recipient,
   wherein the current health status report is a training report of the nursing recipient on the current day, and the training report includes at least one of brain activity test results and walking ability test results,
   wherein the brain activity test results include at least one of brain activity test scores of the corresponding nursing recipient, ranking of brain activity of the corresponding nursing recipient among all nursing recipients, and the name, age, gender and brain activity test scores of all the nursing recipients sorted according to the brain activity test scores,
   wherein the walking ability test results include at least one of walking ability test scores of the corresponding nursing recipient, ranking of walking ability of the corresponding nursing recipient among all nursing recipients, and the name, age, gender and walking ability test scores of all the nursing recipients sorted according to the walking ability test scores,
   wherein the future health status report is a predictive analysis report of detection data of the nursing recipient in a future prescribed period, and the predictive analysis report includes at least one of a brain activity trend, a walking ability trend, a summary of analysis results, and a recommended training menu,
   wherein the management report includes a current management report indicating the current management status of the nursing institution and/or a future management report predicting the future management status of the nursing institution.
- Technical solution 9 is a report generating device, comprising:
   a first memory for storing detection data related to the physical status of a nursing recipient obtained by detecting the body of the nursing recipient; and
   a first data analysis device for generating, according to the detection data, evaluation indexes based on international evaluation standards, i.e., international evaluation standard data, and generating a nursing report for the nursing recipient by analyzing the international evaluation standard data, the international evaluation standards being standards for evaluating human health status.
- The report generating device of technical solution 10, further comprising:
   a second memory for storing management status data indicating the management status of a nursing institution and insurance data analyze indicating a health insurance that the nursing recipient is enrolled in; and
   a second data analysis device for generating a management report suitable for the nursing institution by analyzing the international evaluation standard data, the management status data, and the insurance data.

The report generating device of the present invention can obtain the same technical effects as those of the technical solutions 1 to 8.

### Brief Description of the Drawings

Figure 1 is a diagram showing a first model applying the nursing support system of the present invention.
Figure 2 is a diagram showing a second model applying the nursing support system of the present invention.
Figure 3 is a diagram showing a third model applying the nursing support system of the present invention.
Figure 4 is a schematic diagram showing a nursing support system according to a first embodiment.
Figure 5 is a schematic diagram showing generation of international evaluation standard data.
Figure 6 is a diagram showing an example of a current health status report in the nursing report.
Figure 7 is a diagram showing an example of a future health status report in the nursing report.
Figure 8 is a diagram showing an example of a management report.
Figure 9 is a schematic diagram showing a nursing support system according to a second embodiment.
Figure 10 is a schematic diagram showing a nursing support system according to a third embodiment.
Figure 11 is a schematic diagram showing a nursing support system according to a fourth embodiment.

### Description of the Embodiments

First, models, i.e., scenarios applying the nursing support system of the present invention are described.

### [First Model]

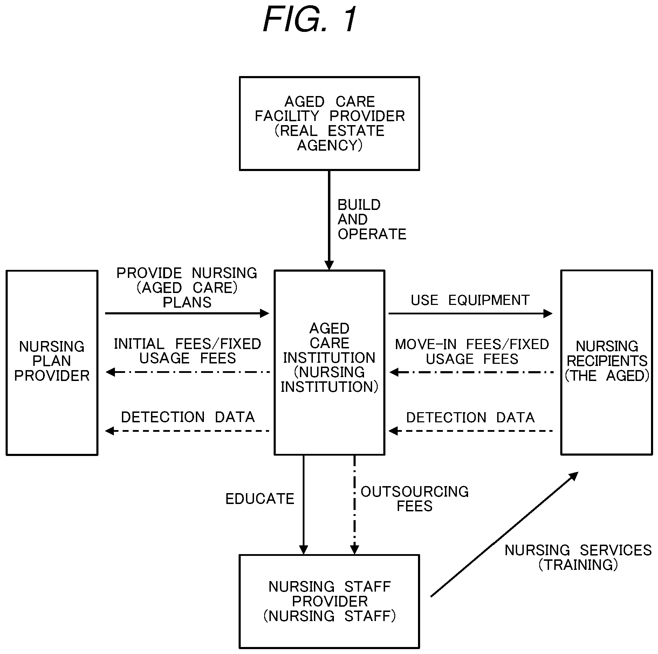

A first scenario, i.e., a first model applying the nursing support system of the present invention will be descried with reference to Figure 1. Figure 1 is a diagram showing a first model applying the nursing support system of the present invention.

As shown in Figure 1, the first model includes a nursing plan provider, an aged care institution, an aged care facility provider, a nursing staff provider, and the aged, i.e., nursing recipients. The aged care institution, such as a nursing home, is equivalent to a nursing institution. The aged care facility provider is, for example, a real estate agency. The nursing staff provider may be either an individual nursing staff or a nursing staff dispatching agency said by which the nursing staff belongs. Hereinafter, for convenience of description, unless otherwise specified, they are collectively referred to as a nursing staff.

### •Nursing plan provider

The nursing plan provider provides the aged care institution with a nursing plan used in the nursing support system of the present invention, an aged care institution management report, a teaching manual for training nursing staffs, etc. In addition, the nursing plan provider receives, from the aged care institution, detection data related to the physical status of nursing recipients, and feedback of the aged care institution on the nursing plan, etc., to learn and update the nursing plan. Moreover, the nursing plan provider collects, from the aged care institution, corresponding fees, i.e., initial fees/fixed usage fees, etc.

### •Aged care facility provider

The aged care facility provider builds and operates aged care facilities required by the aged care institution, such as aged care apartments.

### •Aged care institution

The aged care institution provides various nursing services for nursing recipients. Specifically, a detection device for detecting the body of a nursing recipient is used to obtain detection data related to the physical status of the nursing recipient, the detection data is provided to the nursing plan provider, and the nursing recipient is nursed by means of a nursing report and a management report obtained from the nursing support system of the present invention, thereby improving the management of the aged care institution. The aged care institution collects, from the nursing recipient, corresponding fees, i.e., move-in fees/fixed usage fees, etc.

The aged care institution employs nursing staffs to provide nursing services for nursing recipients. The aged care institution trains the nursing staffs on nursing services by referring to, for example, teaching manuals, etc. The aged care institution pays the nursing staffs for employment.

### •Nursing staff

The nursing staff nurses the nursing recipient according to the nursing report obtained from the nursing support system of the present invention, for example, provides brain training, physical training, etc.

### • Nursing recipient

The nursing recipient receives nursing services from the aged care institution and the nursing staff.

### [Second Model]

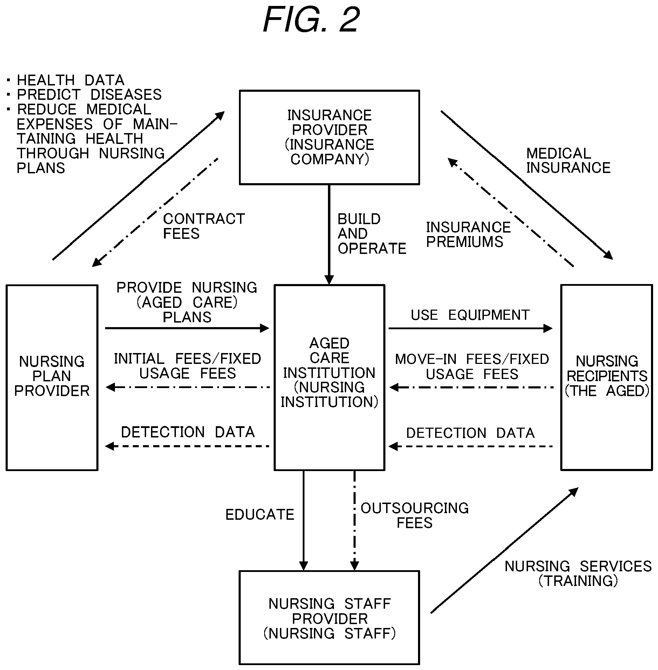

Next, a second scenario, i.e., a second model applying the nursing support system of the present invention will be descried with reference to Figure 2. Figure 2 is a diagram showing a second model applying the nursing support system of the present invention.

As shown in Figure 2, the second model includes a nursing plan provider, an aged care institution, an insurance provider, a nursing staff provider, and the aged, i.e., nursing recipients. The main difference between the second model and the first model lies in that the insurance provider replaces the aged care facility provider in the second model. The insurance provider is, for example, an insurance company. Hereinafter, the content related to the insurance provider will be mainly described, and the description of the same content as that of the first model will be omitted.

### •Nursing plan provider

The nursing plan provider, different from the first model, provides the insurance provider with detection data related to the physical status of a nursing recipient, and future health status, i.e., disease prediction information, of the nursing recipient obtained by the nursing support system of the present invention, and collects corresponding contract fees from the insurance provider.

### • Insurance provider

The insurance provider is responsible for building and operating an aged care apartment. The insurance provider also provides medical insurance to the nursing recipient and collects medical insurance premiums.

In addition, the insurance provider receives from the nursing plan provider the detection data related to the physical status of the nursing recipient, and the future health status, i.e., disease prediction information, of the nursing recipient obtained by the nursing support system of the present invention, and pays the nursing plan provider the corresponding contract fees.

### [Third Model]

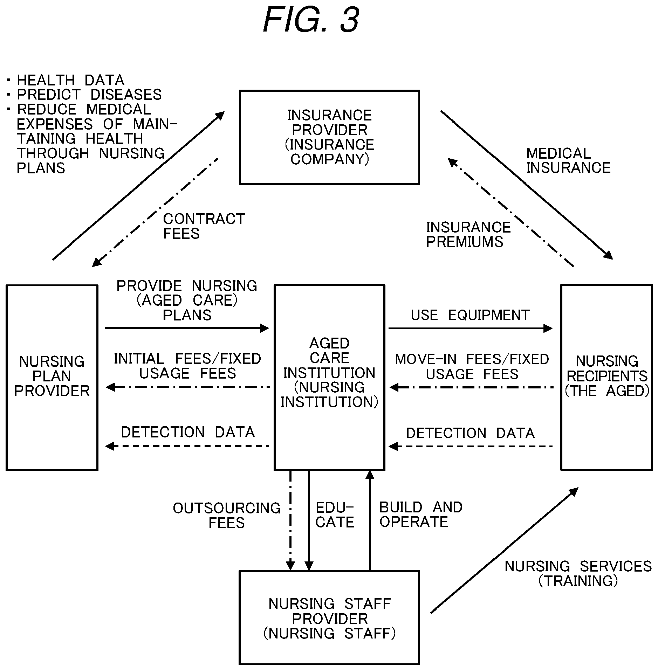

Next, a third scenario, i.e., a third model applying the nursing support system of the present invention will be descried with reference to Figure 3.

As shown in Figure 3, like the second model, the third model includes a nursing plan provider, an aged care institution, an insurance provider, a nursing staff provider, and the aged, i.e., nursing recipients. The third model mainly differs from the second model in that the nursing staff provider provides an aged care apartment.

### •Nursing plan provider

Like the first model, the nursing plan provider provides the aged care institution with a nursing plan used in the nursing support system of the present invention, an aged care institution management report, a teaching manual for training nursing staffs, etc. In addition, the nursing plan provider receives, from the aged care institution, detection data related to the physical status of nursing recipients, feedback of the aged care institution on the nursing plan, etc., to learn and update the nursing plan. Moreover, the nursing plan provider collects, from the aged care institution, corresponding fees, i.e., initial fees/fixed usage fees, etc.

In addition, like the second model, the nursing plan provider provides the insurance provider with detection data related to the physical status of a nursing recipient, and future health status, i.e., disease prediction information, of the nursing recipient obtained by the nursing support system of the present invention, and collects corresponding contract fees from the insurance provider.

### • Insurance provider

Like the second model, the insurance provider provides medical insurance to the nursing recipient and collects medical insurance premiums. In addition, with the insurance provider receives from the nursing plan provider the detection data related to the physical status of the nursing recipient, and the future health status, i.e., disease prediction information, of the nursing recipient obtained by the nursing support system of the present invention, and pays the nursing plan provider the corresponding contract fees.

### •Aged care institution

Like the first model and the second model, the aged care institution provides various nursing services for nursing recipients. Specifically, a detection device for detecting the body of a nursing recipient is used to obtain detection data related to the physical status of the nursing recipient, the detection data is provided to the nursing plan provider, and the nursing recipient is nursed by means of a nursing report and a management report obtained from the nursing support system of the present invention, thereby improving the management of the aged care institution. The aged care institution collects, from the nursing recipient, corresponding fees, i.e., move-in fees/fixed usage fees, etc.

The aged care institution employs nursing staffs from the nursing staff provider to provide nursing services for nursing recipients. The aged care institution trains the nursing staffs on nursing services by referring to, for example, teaching manuals, etc. The aged care institution pays the nursing staffs for employment.

### •Nursing staff provider

Different from the first model and the second model, the nursing staff provider is responsible for building and operating an aged care apartment in the third model. In addition, the nursing staff employed by the aged care institution nurses the nursing recipient according to the nursing report obtained from the nursing support system of the present invention, for example, provides brain training, physical training, etc.

### •Nursing recipient

The nursing recipient receives nursing services from the aged care institution and the nursing staff.

Hereinafter, specific embodiments of the nursing support system of the present invention will be described with reference to the accompanying drawings.

### [First Embodiment]

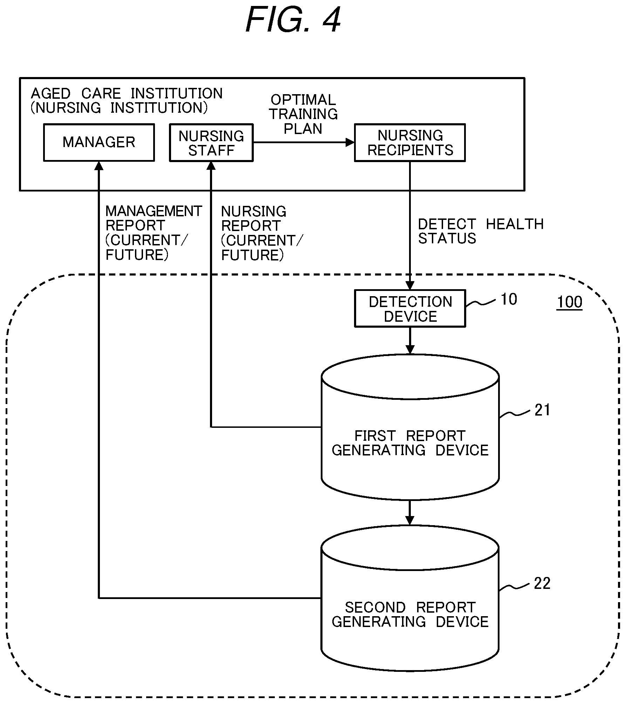

A nursing support system according to a first embodiment will be described with reference to Figure 4.

Figure 4 is a schematic diagram showing a nursing support system according to a first embodiment. As shown in Figure 4, the nursing support system 100 includes a detection device 10, a first report generating device 21, a second report generating device 22, and an output device not shown. The first report generating device and the second report generating device are equivalent to the report generating device in the claims.

The detection tool 10 is a device that detects the body of a nursing recipient to obtain detection data of the physical status of the nursing recipient, and may be, for example, a digital detection tool. The detection data includes test data, vital sign data and basic data. Among them, the test data includes, for example, computing ability scores indicating the mathematical computing ability of the nursing recipient, Oxy-HB (oxyhemoglobin) variation, walking speed, average stride and average acceleration. The vital sign data includes, for example, maximum blood pressure, minimum blood pressure, pulses, blood glucose level, body temperature, sleep state, etc. The basic data includes, for example, gender, age, height, weight, diseases under treatment, fall experience, etc.

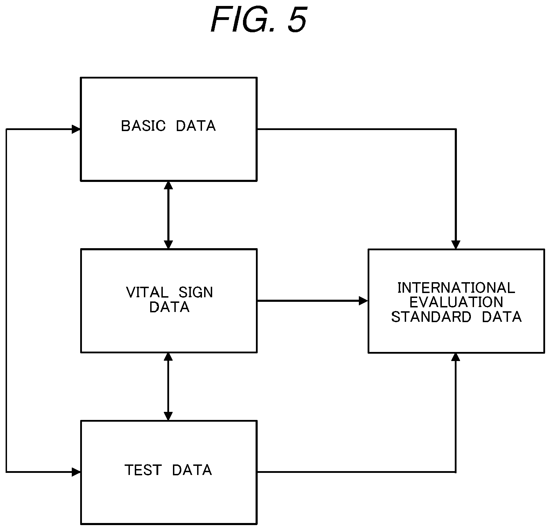

The first report generating device 21 generates, according to the detection data obtained by the detection tool 10, evaluation indexes based on international evaluation standards for the nursing recipient, i.e., international evaluation standard data. Figure 5 is a schematic diagram showing generation of international evaluation standard data. In addition, the first report generating device 21 generates a nursing report suitable for the nursing recipient by analyzing the international evaluation standard data.

The aforementioned international evaluation standards are standards for evaluating human health status, including MMSE (Minimum Mental State Examination), FAB (Frontal Assessment Battery), TMT (Trail Making Test), TUG (Timed Up & Go Test), etc.

Regarding the method of generating the international evaluation standard data according to the detection data obtained by the detection tool 10, a corresponding table between detection data and international evaluation standard data can be used to find the international evaluation standard data corresponding to the detection data therefrom. A relational expression representing the correlation between detection data and international evaluation standard data can also be used, and the detection data is substituted into the relational expression to calculate the international evaluation standard data. A mathematical model composed of detection data and international evaluation standard data can also be constructed by machine learning, to generate the international evaluation standard data from the detection data. For example, the analysis values of MMSE and the like can be inferred by using a known machine learning model such as a Linear Regression (LR) model or a Support Vector Regression (SVR) model. The above is only an example of generating the international evaluation standard data from the detection data, but it is not limited thereto.

The nursing report is a current health status report indicating the current health status of the nursing recipient, a future health status report predicting a trend of the future health status of the nursing recipient, etc. The nursing report is detailed below.

The detection data and the nursing report including the future health status of the nursing recipient can be provided to the insurance plan provider by the nursing plan provider in the aforementioned second model and third model.

The second report generating device 22 obtains the international evaluation standard data from the first report generating device 21, and analyzes the international evaluation standard data, management status data indicating the management status of a nursing institution, and the insurance data indicating a health insurance that the nursing recipient is enrolled in, to generate a management report suitable for the management of the nursing institution. The management report may be a current management report indicating the current management status of the aged care institution, a future management report predicting the future management status of the aged care institution, etc. The management report is detailed below.

The output device outputs the nursing report and the management report generated by the first report generating device 21 and the second report generating device 22, so that the user can use the nursing report and the management report. The output device may be connected to the first report generating device 21 and the second report generating device 22 by cables, or may be remotely connected to the first report generating device 21 and the second report generating device 22 by the Internet or the like. The output device may be, for example, a display device, or a display panel of a mobile terminal or the like.

Table 1 shows data related to the health status of nursing recipients, including detection data such as basic data, vital sign data and test data, and international evaluation standard data based on international evaluation standards. The basic data includes, for example, gender, age, height, weight, etc. The vital sign data includes maximum blood pressure, minimum blood pressure, pulses, blood glucose level, etc. The test data includes the aforementioned computing ability scores, Oxy-HB variation, walking speed, average stride and average acceleration. The international evaluation standard data includes the aforementioned MMSE, FAB, TMT, TUG, etc.

**Table 1**

| No. | BASIC DATA | | | | VITAL SIGN DATA | | | | TEST DATA | | | | | INTERNATIONAL EVALUATION STANDARD DATA | | | |
|---|---|---|---|---|---|---|---|---|---|---|---|---|---|---|---|---|---|
| | GENDER | AGE | HEIGHT | WEIGHT | MAXIMUM BLOOD PRESSURE | MINIMUM BLOOD PRESSURE | PULSE | BLOOD GLUCOSE LEVEL | COMPUTING ABILITY SCORE | Oxy-Hb VARIATION | WALKING SPEED | AVERAGE STRIDE | AVERAGE ACCELERATION | MMSE | FAB | TMT | TUG |
| | - | YEARS OLD | cm | kg | mmHg | mmHg | TIMES/MIN | mg/dL | POINT (s) | - | m/s | m | m/s^2 | - | - | - | - |
| 1 | MALE | 68 | 166 | 70 | 165 | 93 | 71 | 118 | 88 | 0.61 | 1.18 | 0.78 | 0.95 | 24 | 17 | 68 | 9 |
| 2 | MALE | 74 | 168 | 68 | 155 | 97 | 62 | 122 | 56 | 0.46 | 1.4 | 0.91 | 0.93 | 24 | 16 | 78 | 11 |
| 3 | MALE | 79 | 169 | 65 | 110 | 91 | 76 | 102 | 88 | 0.67 | 1.03 | 0.78 | 0.84 | 21 | 12 | 69 | 8 |
| 4 | MALE | 76 | 171 | 70 | 115 | 73 | 71 | 80 | 69 | 0.67 | 1.15 | 0.47 | 1 | 20 | 11 | 70 | 11 |
| 5 | MALE | 78 | 168 | 61 | 115 | 75 | 75 | 91 | 70 | 0.45 | 1.02 | 0.71 | 0.88 | 20 | 17 | 89 | 9 |
| 6 | MALE | 72 | 165 | 69 | 152 | 90 | 80 | 109 | 94 | 0.81 | 0.97 | 0.69 | 1.08 | 19 | 18 | 57 | 6 |
| 7 | MALE | 80 | 173 | 59 | 142 | 91 | 80 | 123 | 92 | 0.8 | 1.15 | 0.95 | 1.04 | 23 | 18 | 81 | 13 |
| 8 | MALE | 75 | 167 | 63 | 119 | 70 | 69 | 81 | 89 | 0.81 | 1.03 | 0.89 | 1.01 | 28 | 10 | 80 | 8 |
| 9 | MALE | 79 | 168 | 61 | 176 | 72 | 77 | 104 | 99 | 0.68 | 1.39 | 0.48 | 1.05 | 30 | 14 | 62 | 12 |
| 10 | MALE | 78 | 160 | 61 | 178 | 95 | 67 | 130 | 96 | 0.88 | 1.35 | 0.58 | 0.8 | 19 | 13 | 97 | 15 |
| 11 | FEMALE | 80 | 155 | 47 | 143 | 88 | 59 | 121 | 71 | 0.9 | 1.36 | 0.55 | 1.18 | 27 | 13 | 54 | 13 |
| 12 | FEMALE | 77 | 148 | 53 | 144 | 94 | 64 | 87 | 56 | 0.62 | 1.19 | 0.59 | 1.13 | 21 | 10 | 93 | 15 |
| 13 | FEMALE | 73 | 150 | 45 | 149 | 94 | 71 | 115 | 95 | 0.89 | 0.82 | 0.79 | 1.04 | 24 | 14 | 55 | 8 |
| 14 | FEMALE | 75 | 147 | 52 | 132 | 98 | 58 | 105 | 85 | 0.5 | 0.99 | 0.56 | 1.09 | 29 | 10 | 66 | 7 |
| 15 | FEMALE | 71 | 161 | 51 | 127 | 97 | 70 | 110 | 100 | 0.66 | 1.11 | 0.92 | 1.09 | 28 | 15 | 75 | 6 |
| 16 | FEMALE | 78 | 145 | 47 | 115 | 82 | 80 | 113 | 86 | 0.74 | 1.4 | 0.85 | 1.14 | 22 | 12 | 61 | 13 |
| 17 | FEMALE | 77 | 158 | 65 | 153 | 94 | 65 | 116 | 57 | 0.4 | 1.29 | 0.5 | 0.96 | 30 | 11 | 94 | 14 |
| 18 | FEMALE | 69 | 162 | 64 | 138 | 99 | 72 | 103 | 59 | 0.75 | 0.74 | 0.87 | 0.84 | 20 | 10 | 77 | 15 |
| 19 | FEMALE | 80 | 154 | 53 | 150 | 95 | 67 | 80 | 90 | 0.54 | 0.72 | 0.88 | 0.8 | 29 | 16 | 67 | 8 |
| 20 | FEMALE | 78 | 163 | 53 | 123 | 98 | 69 | 102 | 94 | 0.45 | 0.95 | 0.88 | 1.1 | 19 | 13 | 71 | 12 |

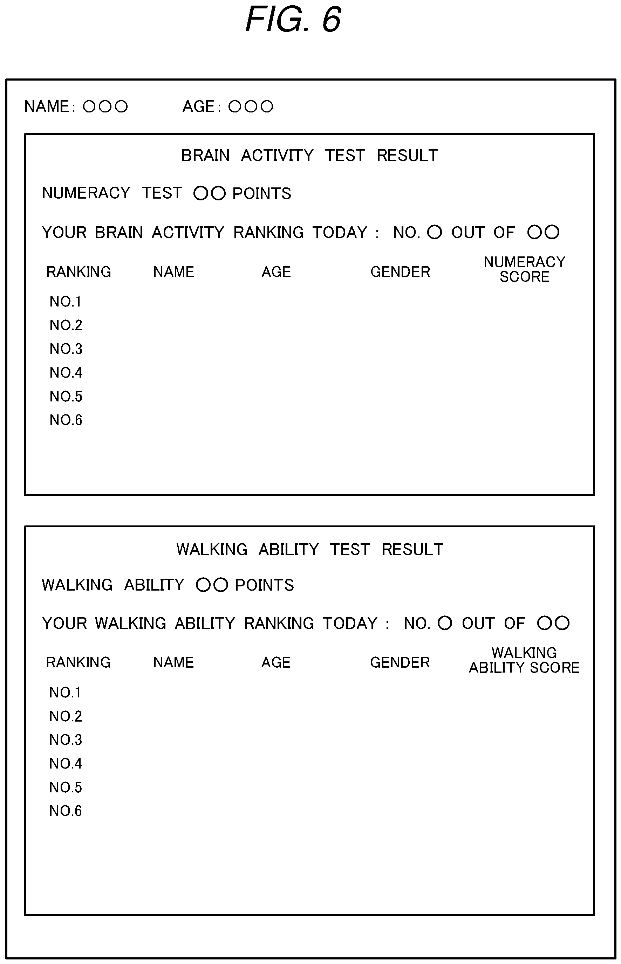

Figure 6 is a diagram showing an example of a current health status report in the nursing report.

The current health status report is a training report of a nursing recipient on the current day, and the training report includes basic information such as the name and age of the nursing recipient.

The training report further includes brain activity test results and walking ability test results.

The brain activity test results include brain activity test scores of the nursing recipient, ranking of brain activity of the nursing recipient among all nursing recipients, mean value comparison with contemporaries, individual detailed training scores, and the name, age, gender and brain activity test scores of all the nursing recipients sorted according to the brain activity test scores.

The walking ability test results include walking ability test scores of the nursing recipient, ranking of walking ability of the nursing recipient among all nursing recipients, mean value comparison with contemporaries, individual detailed training scores, and the name, age, gender and walking ability test scores of all the nursing recipients sorted according to the walking ability test scores.

The current health status report in Figure 6 is only an example, and the current health status report is not limited thereto. For example, the ranking of the brain activity and walking ability of the nursing recipient among all nursing recipients is shown in Figure 6, but results of comparing the brain activity and walking ability of the nursing recipient with the mean values of contemporaries can also be shown by a radar chart.

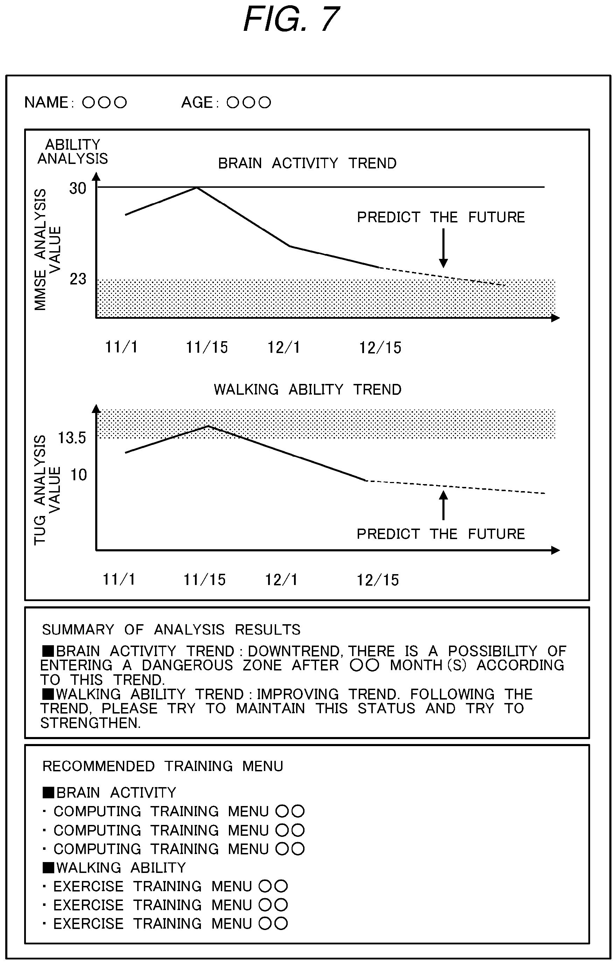

Figure 7 is a diagram showing an example of a future health status report in the nursing report.

The future health status report is a predictive analysis report of detection data of the nursing recipient in a future prescribed period, and the future health status report includes basic information of the nursing recipient such as name and age.

The future health status report further includes a brain activity trend, a walking ability trend, a summary of analysis results, and a recommended training menu for the nursing recipient.

In the graph showing the brain activity trend in Figure 7, the horizontal axis represents date, the vertical axis represents MMSE (Minimum Mental State Examination) analysis value, the solid line represents past MMSE analysis value of the nursing recipient, the dotted line represents predicted future MMSE analysis value of the nursing recipient, and the gray area indicates a bad data area. Regarding the setting of a threshold value of the bad data area of the MMSE analysis value, generally, the MMSE analysis value of 27 scores or less is considered as suspected mild cognitive impairment (MCI), and the MMSE analysis value of 23 scores or less is considered as suspected cognitive impairment. In the example of Figure 7, the MMSE analysis value of 23 scores is set as the threshold value, and the area of 23 scores or less is set as the bad data area. The threshold value of the bad data area of the MMSE analysis value can also be appropriately adjusted to other values.

Similarly, in the graph showing the walking ability trend in Figure 7, the horizontal axis represents date, the vertical axis represents TUG (Timed Up & Go Test) analysis value, the solid line represents past TUG analysis value of the nursing recipient, the dotted line represents predicted future TUG analysis value of the nursing recipient, and the gray area indicates a bad data area. Regarding the setting of a threshold value of the bad data area of the TUG analysis value, the TUG analysis value of more than 13.5 seconds is generally considered as high risk of falling.

In the example of Figure 7, the TUG analysis value of 13.5 seconds is set as the threshold value, and the area of more than 13.5 seconds is set as the bad data area. The threshold value of the bad data area of the TUG analysis value can also be appropriately adjusted to other values.

The summary of analysis results record analysis on the brain activity trend and analysis on the walking ability trend. The analysis on the brain activity trend is, for example, "Downtrend. There is a possibility of entering a dangerous zone after ∘∘ month(s) according to this trend". The analysis on the walking ability trend is, for example, "Improving trend. Following the trend, please try to maintain this status and try to strengthen".

In the recommended training menu, future training content suitable for the nursing recipient is provided according to the summary of analysis results of the nursing recipient. For example, a corresponding table about types of international evaluation standard data, values of international evaluation standard data or variations of international evaluation standard data before and after training, and training items may be pre-saved, and corresponding training items or training intensity may be recommended according to the predicted values of the international evaluation standard data or the predicted variations of the international evaluation standard data before and after training.

The future health status report in Figure 7 is only an example, and the future health status report is not limited thereto.

Returning to Figure 4, the nursing staff grasps the health status of the nursing recipient from the nursing report output by the output device, and provides nursing services such as health training to the nursing recipient.

Specifically, the current health status of the nursing recipient such as brain activity and walking ability, the ranking of the health status of the nursing recipient among all nursing recipients, the comparison with the average health value of contemporaries, etc. can be grasped according to the current health status report.

In addition, the trend of the health status such as the brain activity and walking ability of the nursing recipient in the past period and the future period can be grasped according to the future health status report, so whether the nursing services such as health training for the nursing recipient in the past period is effective can be grasped. Moreover, the nursing services such as health training in the future period can be reasonably arranged according to the recommended training menu suggested by the future health status report.

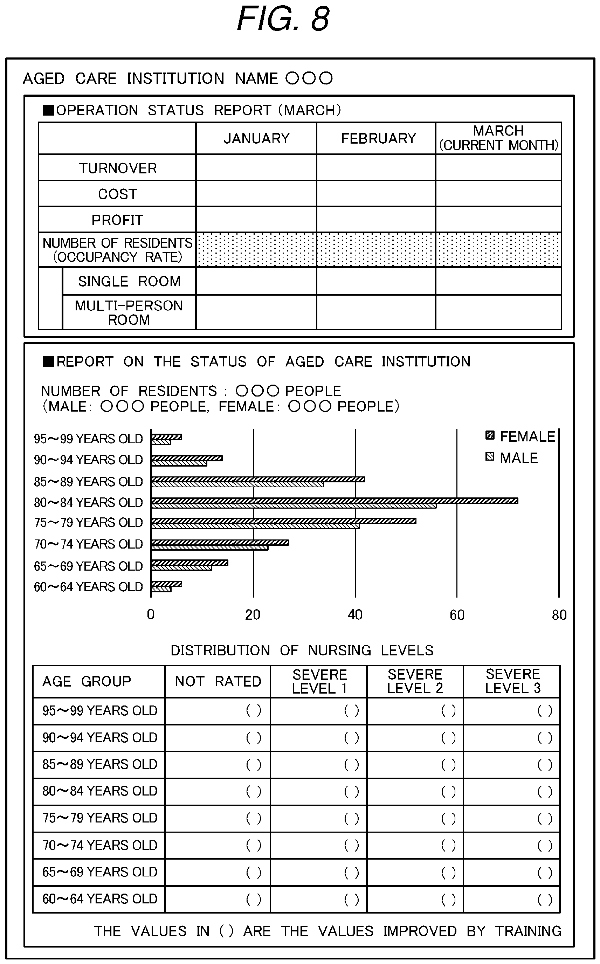

Figure 8 is a diagram showing an example of a management report generated by the second report generating device 22.

The management report includes basic information such as the name of an aged care institution, a management status report, and an aged care institution status report.

Taking March as an example, the management status report includes financial information such as turnover, cost, and profit in January, February, and March, as well as information such as the number of occupants (occupancy rate), among which the number of occupants (occupancy rate) can also be subdivided according to single rooms and multi-person rooms.

In the aged care institution status report, the basic information of nursing recipients is represented by ages and genders. For example, how many men and women are in each age group of 60-64 years old, 65-69 years old, 70-74 years old, 75-79 years old, 80-84 years old, 85-89 years old, 90-94 years old, and 95-99 years old.

In addition, the aged care institution status report further includes distribution information of nursing levels required by the nursing recipients. For example, the number of people at different nursing levels is represented by ages. Specifically, the number of nursing recipients at non-correspondence, severity level 1, severity level 2, and severity level 3 are respectively represented by 60-64 years old, 65-69 years old, 70-74 years old, 75-79 years old, 80-84 years old, 85-89 years old, 90-94 years old, and 95-99 years old. According to the data of each nursing recipient, the current number of nursing recipients at each nursing level can be counted, to predict the number of nursing recipients at each nursing level in a future period. For example, according to the stored personal data, training (rehabilitation) status, etc. of each nursing recipient, the number of nursing recipients corresponding to non-correspondence, severity level 1, severity level 2, and severity level 3 can be represented, and the predicted number of nursing recipients corresponding to non-correspondence, severity level 1, severity level 2, and severity level 3 after 1 year of training can also be represented. For example, the number of nursing recipients at non-correspondence, severity level 1, severity level 2, and severity level 3 after training and improvement can be filled in brackets for comparative analysis.

The management report in Figure 8 is only an example, and the management report is not limited thereto. In addition, although any specific numerical value is not recorded in Figure 8, specific numerical values are recorded in an actual management report. In addition, the management report in Figure 8 is a report showing the current management status of an aged care institution. Although not shown, the second report generating device 22 can also generate a future management report predicting the future management status of the aged care institution, and the future management report reaction the trend of the management status under the circumstance that the management plan so far is continued, for example, whether the management status will get worse or better.

Returning to Figure 4, the manager of the aged care institution grasps the management status of the aged care institution from the management report output by the output device, and makes a future management plan of the aged care institution.

Specifically, the current management status of the aged care institution and whether the management plan implemented in the past period is suitable for the aged care institution can be grasped according to the current management report. In addition, the age distribution, gender distribution, and required nursing levels of the nursing recipients in the aged care institution can also be grasped. Therefore, a future management plan of the aged care institution can be made according to the current management status and the conditions of the nursing recipients.

Moreover, whether the management plan currently being implemented is reasonable can be grasped according to the future management report, and the management plan is maintained or improved accordingly.

Next, the technical effects of the nursing support system of the first embodiment will be described.

The understanding of the health status of nursing recipients by questionnaires in the past inevitably relies on the subjective consciousness of the nursing recipients, and the nursing recipients in the same health status may provide different answers, resulting in deviations and failure to accurately grasp the health status of the nursing recipients.

Contrary to this, in the nursing support system of the first embodiment, when the health status of a nursing recipient is acquired, the detection device acquires detection data of the nursing recipient, including test data such as computing ability scores, Oxy-HB variation, walking speed, average stride and average acceleration, vital sign data such as maximum blood pressure, minimum blood pressure, pulses and blood glucose level, and basic data such as gender, age, height and weight, and these detection data are all objective data and do not rely on the subjective consciousness of the nursing recipient. Therefore, the international evaluation standard data generated according to these objective detection data can objectively and accurately reflect the health status of the nursing recipient. Therefore, the nursing staff can accurately grasp the health status of the nursing recipient, and provide targeted nursing services to the nursing recipient accordingly.

In addition, the past method of questionnaire cannot predict the trend of the future health status of the nursing recipient.

Contrary to this, the nursing support system of the first embodiment can generate a future health status report predicting the future health status of the nursing recipient. Thus, the nursing staff can not only grasp the past health status of the nursing recipient, but also know the trend of the future health status, and therefore can reasonably arrange future nursing plans such as health training.

In addition, the nursing support system of the first embodiment generates a management report of the aged care institution by analyzing the international evaluation standard data, the management status data, and the insurance data. The international evaluation standard data, the management status data, and the insurance data are all objective data, and thus the objective management report can be provided for the manager of the aged care institution, to accurately grasp the current management status and make a future management plan of the aged care institution according to the current management status and the status of the nursing recipient.

In addition, the nursing support system of the first embodiment can further generate a future management report of the aged care institution, thereby reducing the man-hours required for the manager of the aged care institution to predict the future management status.

### [Second Embodiment]

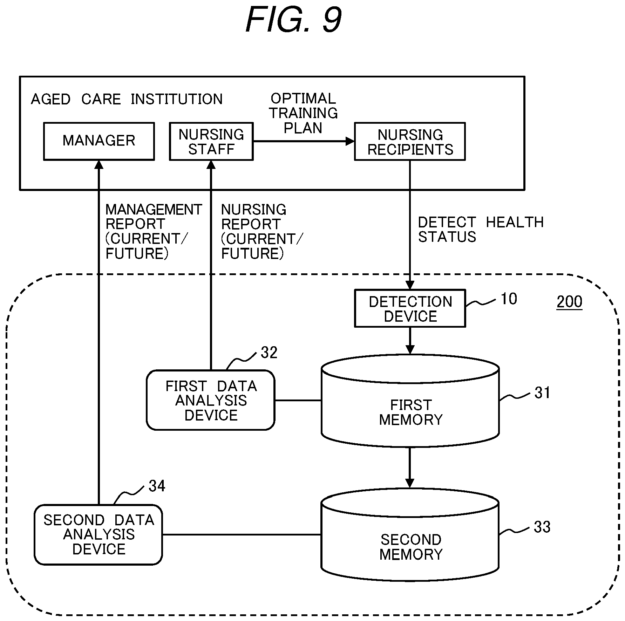

A nursing support system according to a second embodiment will be described with reference to Figure 9.

Figure 9 is a schematic diagram showing a nursing support system 200 according to a second embodiment. In the second embodiment, the same reference numerals are assigned to the same parts as those of the first embodiment, and the description of the same parts as those of the first embodiment is omitted.

As shown in Figure 9, the nursing support system 200 includes a detection device 10, a first memory 31, a first data analysis device 32, a second memory 33, a second data analysis device 34, and an output device not shown.

The combination of the first memory 31 and the first data analysis device 32 in the second embodiment is equivalent to the first report generating device 21 in the first embodiment, and the combination of the second memory 33 and the second data analysis device 34 in the second embodiment is equivalent to the second report generating device 22 in the first embodiment.

The first memory 31 stores detection data related to the physical status of a nursing recipient obtained by detecting the body of the nursing recipient. The detection data is the same as that in the first embodiment, so detailed description is omitted.

The first data analysis device 32 generates, according to the detection data, evaluation indexes of a nursing recipient based on international evaluation standards, i.e., international evaluation standard data, and generates a nursing report suitable for the nursing recipient by analyzing the international evaluation standard data. The nursing report is a current health status report indicating the current health status of the nursing recipient, a future health status report predicting a trend of the future health status of the nursing recipient, etc. The nursing report is the same as that in the first embodiment, so detailed description is omitted.

The detection data and the nursing report including the future health status of the nursing recipient can be provided to the insurance plan provider by the nursing plan provider in the aforementioned second model and third model.

The second memory 33 stores management status data indicating the management status of a nursing institution and insurance data indicating a health insurance that the nursing recipient is enrolled in; and

The second data analysis device generates a management report suitable for the management of the nursing institution by analyzing the international evaluation standard data generated by the first data analysis device, the management status data, and the insurance data. The management report may be a current management report indicating the current management status of the aged care institution, a future management report predicting the future management status of the aged care institution, etc. The management report is the same as that in the first embodiment, so detailed description is omitted.

Like the first embodiment, the output device outputs the nursing report and the management report generated by the first data analysis device 32 and the second data analysis device 34, so that the user can use the nursing report and the management report. The output device may be connected to the first data analysis device 32 and the second data analysis device 34 by cables, or may be remotely connected to the first data analysis device 32 and the second data analysis device 34 by the Internet or the like.

The second embodiment can obtain the same technical effects as those in the first embodiment.

### [Third Embodiment]

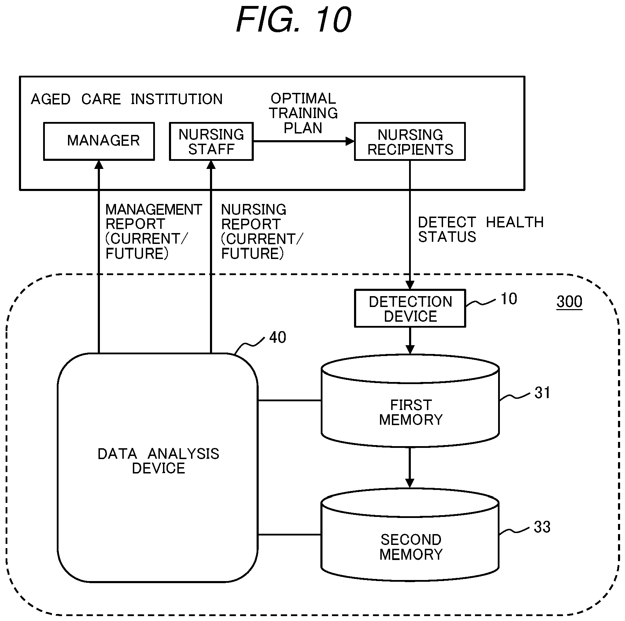

A nursing support system according to a third embodiment will be described with reference to Figure 10.

Figure 10 is a schematic diagram showing a nursing support system 300 according to a third embodiment. In the third embodiment, the same reference numerals are assigned to the same parts as those of the first embodiment and the second embodiment, and the description of the same parts is omitted.

As shown in Figure 10, the nursing support system 300 includes a detection device 10, a first memory 31, a second memory 33, a data analysis device 40, and an output device not shown.

The data analysis device 40 in the third embodiment is equivalent to the combination of the first data analysis device 32 and the second data analysis device 34 in the second embodiment.

The data analysis device 40 generates, according to the detection data, evaluation indexes of a nursing recipient based on international evaluation standards, i.e., international evaluation standard data, and generates a nursing report suitable for the nursing recipient by analyzing the international evaluation standard data. The nursing report is a current health status report indicating the current health status of the nursing recipient, a future health status report predicting a trend of the future health status of the nursing recipient, etc. The nursing report is the same as those in the first embodiment and the second embodiment, so detailed description is omitted.

The detection data and the nursing report including the future health status of the nursing recipient can be provided to the insurance plan provider by the nursing plan provider in the aforementioned second model and third model.

The data analysis device 40 further generates a management report suitable for the management of the nursing institution by analyzing the international evaluation standard data, the management status data, and the insurance data. The management report may be a current management report indicating the current management status of the aged care institution, a future management report predicting the future management status of the aged care institution, etc. The management report is the same as those in the first embodiment and the second embodiment, so detailed description is omitted.

Like the first embodiment and the second embodiment, the output device outputs the nursing report and the management report generated by the data analysis device 40, so that the user can use the nursing report and the management report. The output device may be connected to the data analysis device 40 by a cable, or may be remotely connected to the data analysis device 40 by the Internet or the like.

The third embodiment can obtain the same technical effects as those in the first embodiment and the second embodiment.

### [Fourth Embodiment]

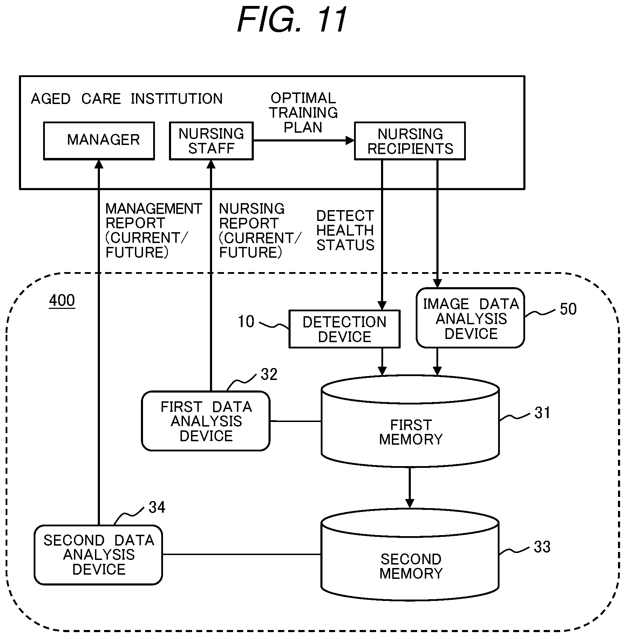

A nursing support system according to a fourth embodiment will be described with reference to Figure 11.

Figure 11 is a schematic diagram showing a nursing support system 400 according to a fourth embodiment. In the fourth embodiment, the same reference numerals are assigned to the same parts as those of the second embodiment, and the description of the same parts is omitted.

As shown in Figure 11, the nursing support system 400 includes a detection device 10, a first memory 31, a first data analysis device 32, a second memory 33, a second data analysis device 34, an image data analysis device 50, and an output device not shown.

The fourth embodiment differs from the second embodiment in the image data analysis device 50 included.

The image data analysis device 50 acquires image data of a nursing recipient from an image capture device such as a camera that captures the stride and the like of the nursing recipient, analyzes the image data, and stores the image analysis data in the first memory 31, and the image analysis data may also be used as detection data.

The first data analysis device 32 generates international evaluation standard data according to the detection data, and generates a nursing report suitable for the nursing recipient by analyzing the international evaluation standard data. The nursing report is the same as those in the first to fourth embodiments, so detailed description is omitted.

The fourth embodiment can obtain the same technical effects as those in the first embodiment and the third embodiment.

Next, the effects that can be produced by the aforementioned first model applying the nursing support system according to the first to fourth embodiments of the present invention will be described.

The nursing support system of the present invention can generate a nursing report for a nursing recipient, and therefore, the aforementioned first model can produce the following effects by applying the nursing support system of the present invention.

For the nursing recipient, since the nursing support system of the present invention can generate a nursing report for the nursing recipient, the nursing recipient can obtain nursing services suitable for himself from the aged care institution where he lives, for example, can obtain services of alleviating cognitive impairment and falling risk, obtain appropriate care from the nursing staff, and enjoy services previously unavailable in the aged care institution. Therefore, the life span can be prolonged, and the sense of security and superiority can be obtained.

For the nursing staff, he can obtain knowledge related to health data from the nursing support system of the present invention by using the detection device provided by the aged care institution, obtain targeted training from the aged care institution, and obtain stable income from the aged care institution. That is, the nursing staff can reserve nursing skills, improve the level of human resources, and obtain stable income.

For the aged care facility provider, the nursing support system of the present invention can generate a nursing report for a nursing recipient, and the nursing recipient can enjoy services previously unavailable in the aged care institution, and affluent nursing recipients can be attracted, to obtain stable income. In addition, since the management report generated by the nursing support system of the present invention can be obtained, stable management can be realized. That is, the aged care facility provider can improve its brand power, gain stable benefits, and even expand the benefits.

Next, the effects that can be produced by the aforementioned second model applying the nursing support system of the present invention will be described.

Since detection data related to the physical status of a nursing recipient and future health status, i.e., disease prediction information of the nursing recipient can be obtained, the health status of the nursing recipient can be practically grasped. In addition, the nursing support system of the present invention can generate a nursing report for a nursing recipient, and the nursing recipient can enjoy services previously unavailable in the aged care institution, and affluent nursing recipients can be attracted, to obtain stable income. In addition, since the management report generated by the nursing support system of the present invention can be obtained, stable management can be realized. In addition, by providing targeted nursing services to the nursing recipient, the incidence of diseases of the nursing recipient can be reduced, therefore, the insurance provider can reduce medical expenses paid to the medical institution.

That is, the insurance provider can improve its brand power, gain stable benefits, and reduce medical expenses.

By applying the nursing support system of the present invention to the third model, the parties in the third model can also obtain the same effects as those of the first model and the second model.

The embodiments of the present invention are described above with reference to the accompanying drawings. The embodiments described above are only specific examples of the present invention, and are used for understanding the present invention, instead of limiting the scope of the present invention. Those skilled in the art can make various modifications, combinations, and reasonable omissions of elements to each embodiment based on the technical ideas of the present invention, and the resulting embodiments also fall within the scope of the present invention.

## Claims

1. A nursing support system, comprising:
a detection device for detecting the body of a nursing recipient to obtain detection data related to the physical status of the nursing recipient;
a first report generating device for generating, according to the detection data, evaluation indexes based on international evaluation standards, i.e., international evaluation standard data, and generating a nursing report for the nursing recipient by analyzing the international evaluation standard data, the international evaluation standards being standards for evaluating human health status; and
an output device for outputting the nursing report.

2. The nursing support system according to claim 1,
further comprising a second report generating device, which analyzes the international evaluation standard data, management status data indicating the management status of a nursing institution, and insurance data indicating a health insurance that the nursing recipient is enrolled in, to generate a management report suitable for the nursing institution.

3. The nursing support system according to claim 2,
wherein the first report generating device includes:
a first memory for storing the detection data; and
a first data analysis device for generating the international evaluation standard data according to the detection data, and generating the nursing report by analyzing the international evaluation standard data,
wherein the second report generating device includes:
a second memory for storing the management status data and the insurance data; and
a second data analysis device for generating the management report by analyzing the international evaluation standard data input by the first data analysis device, the management status data, and the insurance data.

4. The nursing support system according to any one of claims 1 to 3,
wherein the nursing report includes a current health status report indicating the current health status of the nursing recipient and/or a future health status report predicting a trend of the future health status of the nursing recipient.

5. The nursing support system according to any one of claims 1 to 3,
wherein the current health status report is a training report of the nursing recipient on the current day, including information on brain activity test scores and walking ability test scores of the nursing recipient, information on the ranking of brain activity and walking ability among all nursing recipients, mean value comparison with contemporaries, and individual detailed training scores.

6. The nursing support system according to any one of claims 1 to 3,
wherein the future health status report is a predictive analysis report of detection data of the nursing recipient in a future prescribed period, including information indicating a brain activity trend, a walking ability trend, a summary of analysis results, and a recommended training menu for the nursing recipient.

7. The nursing support system according to claim 2 or 3,
wherein the management report includes a current management report indicating the current management status of the nursing institution and/or a future management report predicting the future management status of the nursing institution, the current management report includes current financial information and distribution information of current nursing levels required by the nursing recipients, and the future management report includes future financial information and distribution information of future nursing levels required by the nursing recipients.

8. The nursing support system according to claim 1 or 2,
wherein the detection device includes a detection tool for detecting the body of the nursing recipient, and the detection tool detects the body of the nursing recipient to obtain detection data related to the physical status of the nursing recipient; the detection data includes test data, vital sign data and basic data; the test data includes at least one of computing ability scores, Oxy-HB (oxyhemoglobin) variation, walking speed, average stride and average acceleration; the vital sign data includes at least one of maximum blood pressure, minimum blood pressure, pulses, and blood glucose level; the basic data includes at least one of gender, age, height, and weight;
wherein the international evaluation standards generated by the first report generating device includes at least one of MMSE (Minimum Mental State Examination), FAB (Frontal Assessment Battery), TMT (Trail Making Test), and TUG (Timed Up & Go Test);
wherein the nursing report includes a current health status report indicating the current health status of the nursing recipient and/or a future health status report predicting a trend of the future health status of the nursing recipient;
wherein the current health status report is a training report of the nursing recipient on the current day, and the training report includes at least one of brain activity test results and walking ability test results;
wherein the brain activity test results include at least one of brain activity test scores of the corresponding nursing recipient, ranking of brain activity of the corresponding nursing recipient among all nursing recipients, and the name, age, gender and brain activity test scores of all the nursing recipients sorted according to the brain activity test scores;
wherein the walking ability test results include at least one of walking ability test scores of the corresponding nursing recipient, ranking of walking ability of the corresponding nursing recipient among all nursing recipients, and the name, age, gender and walking ability test scores of all the nursing recipients sorted according to the walking ability test scores;
wherein the future health status report is a predictive analysis report of detection data of the nursing recipient in a future prescribed period, and the predictive analysis report includes at least one of a brain activity trend, a walking ability trend, a summary of analysis results, and a recommended training menu; the management report includes a current management report indicating the current management status of the nursing institution and/or a future management report predicting the future management status of the nursing institution.

9. A report generating device, comprising:
a first memory for storing detection data related to the physical status of a nursing recipient obtained by detecting the body of the nursing recipient; and
a first data analysis device for generating, according to the detection data, evaluation indexes based on international evaluation standards, i.e., international evaluation standard data, and generating a nursing report for the nursing recipient by analyzing the international evaluation standard data, the international evaluation standards being standards for evaluating human health status.

10. The report generating device according to claim 9, further comprising:
a second memory for storing management status data indicating the management status of a nursing institution and insurance data analyze indicating a health insurance that the nursing recipient is enrolled in; and
a second data analysis device for generating a management report suitable for the nursing institution by analyzing the international evaluation standard data, the management status data, and the insurance data.
